# EUROPEAN PATENT SPECIFICATION

(11) **EP 1 247 538 B1**
(45) Date of publication and mention of the grant of the patent: **24.08.2005**
(21) Application number: 02007100.7
(22) Date of filing: 28.03.2002
(51) Int. Cl.: A61M 5/142

(54) **Disposable tubing set for liquid pump**
Einmalschlauchset für Flüssigkeitspumpe
Ensemble de tuyaux jetables pour pompe à liquide

(43) Date of publication of application: 09.10.2002
(73) Proprietor: Barak, Swi, Caesarea 38900 (IL)
(72) Inventor: Barak, Swi, Caesarea 38900 (IL)
(74) Representative: Möbus, Daniela

(56) References cited:
- EP-A- 0 526 920
- WO-A-01/64265
- WO-A-98/56453
- US-A- 4 798 590
- US-A- 4 820 281
- US-A- 5 634 907
- US-A- 5 954 485

## Description

### FIELD AND BACKGROUND OF THE INVENTION

This invention relates to liquid administration and more specially, it is concerned with a liquid flow set useful for administration of liquids to a patient through a flexible tube.

Systems for administration of liquids to a patient are widely known. However, a variety of different pumps are available for propelling a liquid to a patient, which may differ, among others, by construction and safety of use.

Flow sets for use with a liquid pump must attentively design for safe use. A dedicated tube-segment of the tube must be installed in the pump and the tube must be installed in the right place, tighten, straight and stretched. The flow set must be full with liquid before using it and should be remain full as long as it in use. Moreover, the pumping tube-segment must replace with other tube-segment when it loss it's flexibility.

From US 4,798,590 a flow set became known, comprising a drip chamber, a flexible plastic line having a roller clamp, an inlet connector coupled to an end of a pump chamber, wherein the other end of the pump chamber is coupled to an outlet connector. The outlet connector is coupled through a line having a roller clamp and a Y-site therein to a luer.

The present invention is concerned with a liquid flow set that includes a number of safety features in order to identify such a flow set by the liquid pump that uses it.

### SUMMARY OF THE INVENTION

The present invention is related to a disposable flow set for administration of liquid by using a liquid pump.

According to the teachings of the present invention there is provided a disposal flow set for administrating liquid including:
(a) a drip chamber or a spike;
(b) a first administration tube to administer liquid from the drip chamber;
(c) at least one pumping tube-segment, connected to the first administration tube, enabling said flow set to be installed in a liquid pump;
(d) a second administration tube installed at the end of the last one of said pumping tube-segments; and
(e) an anti-free-flow valve installed at the end of the second administration tube.
   The flow set further includes:
(f) at least one identifying-key, whereby the or each pumping tube-segment has a respective identifying-key connected thereto, and wherein each identifying-key comprises a hoob that is clamped on the respective tube-segment and includes:
   (i) a number of teeth with a unique combination of location and width, these teeth are to be insert into a compatible niches in a specific liquid pump and prevents insertion of the flow set to a non compatible liquid pump;
   (ii) a pressing-plate for being pressed by a door (25) of a liquid pump (17) in order to allow the tube-segment to be pressed against a pressure sensor of said liquid pump while the door of the liquid pump is closed enabling said liquid pump to use the sensed pressure to verify the existence of the flow set.

By another preferred embodiment each pumping tube-segment of the disposal flow set is bordered by a stopper in both ends, these stoppers are shaped to install in fitted niches in a liquid pump.

By another preferred embodiment the length of each pumping tube-segment of the disposal flow set is between 81 millimeter and 83 millimeter.

By another preferred embodiment the inside diameter of the pumping tube-segments of the disposal flow set is between 2.5 millimeter and 2.7 millimeter and the diameter of the first and/or second administration tube is between 1.1 millimeter and 1.3 millimeter.

By another preferred embodiment the disposal flow set further includes a controlling means in the inlet of the drip chamber, enabling the control of the volume of the entering drops.

### BRIEF DESCRIPTION OF THE FIGURES

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making it apparent to those skilled in the art how the several forms of the invention may be embodied in practice.

In the figures:
Figure 1 illustrates a flow set with drip chamber, a number of pumping tube-segments and anti-free-flow valve.
Figure 2 illustrates the way to install a pumping tube-segment in a liquid pumping.
Figure 3 illustrates a cross section of an identifying-key.
Figure 4 illustrates a chart of the pressure inside the tube of a flow set during the priming procedure.

### DESCRIPTION OF THE PREFERED EMBODIMENTS

The present invention is a flow set for administration of liquid when using a liquid pump, and for identification by a liquid pump that uses this flow set.

Flow sets for use with a liquid pump must attentively be designed for safe use. A dedicated tube-segment of the tube must be installed in the pump and the tube must be installed in the right place, tightened, straight and stretched. The flow set must be full with liquid before using it and should be remain full as long as it is in use. Moreover, the pumping tube-segment must be replaced by another tube-segment when it losses its' flexibility.

The flow set of the present invention has a number of features that ensures the use of only this specific flow set in a compatible liquid pump. There are stoppers at both ends of pumping tube-segments that are shaped to be installed in fitted niches in the liquid pump, and there is an identifying-key that prevents the insertion of a wrong flow set and enables liquid pressure measuring - without this measuring the liquid pump will not start. Each pumping tube-segment has a specific length.

The principles and operation of the flow set according to the present invention, and the use of the flow set, may be better understood with reference to the drawings and the accompanying description.

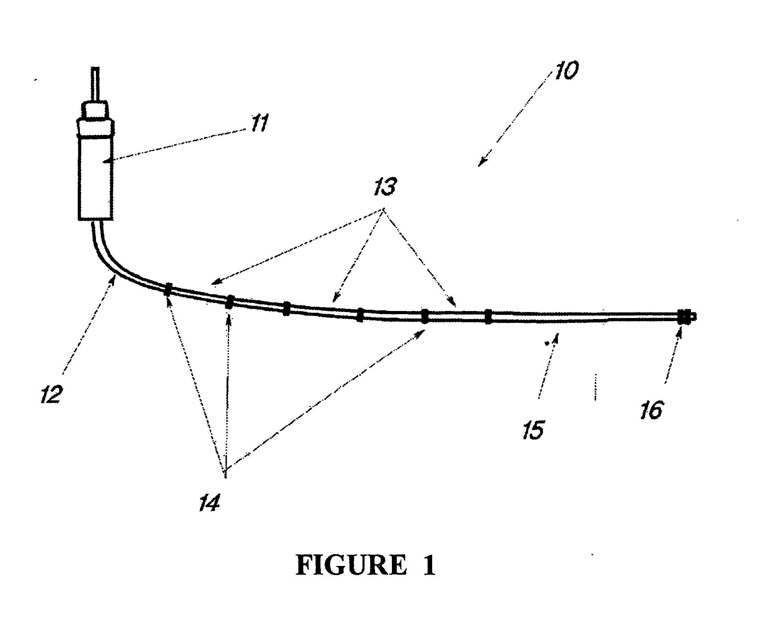

Referring now to the drawings. Figure 1 illustrates a flow set with a drip chamber, a number of pumping tube-segments and anti-free-flow valve. A flow set **10** includes a drip chamber **11** enabled to be installed in a housing with drop sensors located in a liquid pump (not shown). The drop sensor counts the drops passing through the drip chamber **11.** A first administration tube **12** administrates the liquid through pumping tube-segments **13.** Stoppers **14** at both ends border each pumping tube-segment **13,** these stoppers **14** are used to install the pumping tube-segment **13** in a liquid pump, in a correct position - tightened, straight and stretched. A second administration tube **15** administrates the pumped liquid to an anti-free-flow valve **16.** An identifying-key **24** is installed on each pumping tube segment **13**.

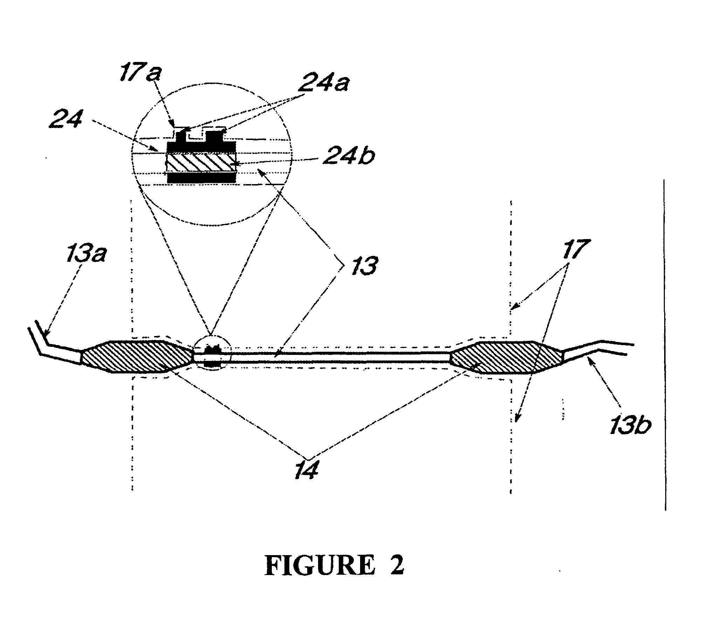

Figure 2 illustrates the way to install a pumping tube-segment in a liquid pump. There are number of pumping tube-segments **13, 13a, 13b** ... each tube-segment is bordered by stoppers **14.** One of the pumping tube-segments **13** is in use and installed in a liquid pump **17.** An identifying-key **24** is installed on each tube-segment **13.** The identifying-key **24** is clamped to the tube-segment **13** and includes a number of teeth **24a** each tooth has a specific width and specific location, creating a code that enables inserting the tube-segment **13** only to a specific liquid pump **17** that has a set of niches **17a** that are arranged in the same code. The identifying-key **24** has also a pressure-plate **24b,** this pressure-plate **24b** is pressed by a door (not shown) of the liquid pump **17** and presses the tube-segment **13** against a pressure sensor (not shown) that is installed in the liquid pump **17** behind the identifying-key **24.** The stoppers **14** are designed to be installed in niches of the liquid pump **17** and force the pumping tube-segment **13** to stay tightened, straight and stretched. The identifying-key **24** prevents insertion of the tube-segment **13** to a non-compatible liquid pump and the liquid pump **17** will not start unless the pressure sensor measures pressure.

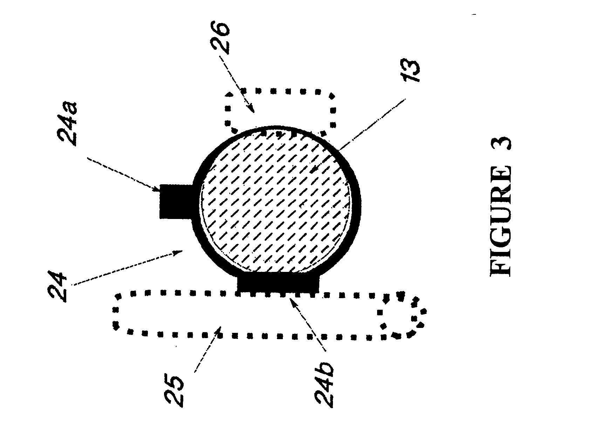

Figure 3 illustrates a cross section of an identifying-key. The identifying-key **24** is clamped to the tube-segment **13.** The identifying-key **24** includes a set of teeth **24a** to enable the insertion of the flow set only into a compatible liquid pump and a pressure-plate **24b**. This pressure-plate **24b** is pressed by a door **25** of the liquid pump and presses the tube-segment **13** against a pressure sensor **26** of the liquid pump enabling the pressure measuring.

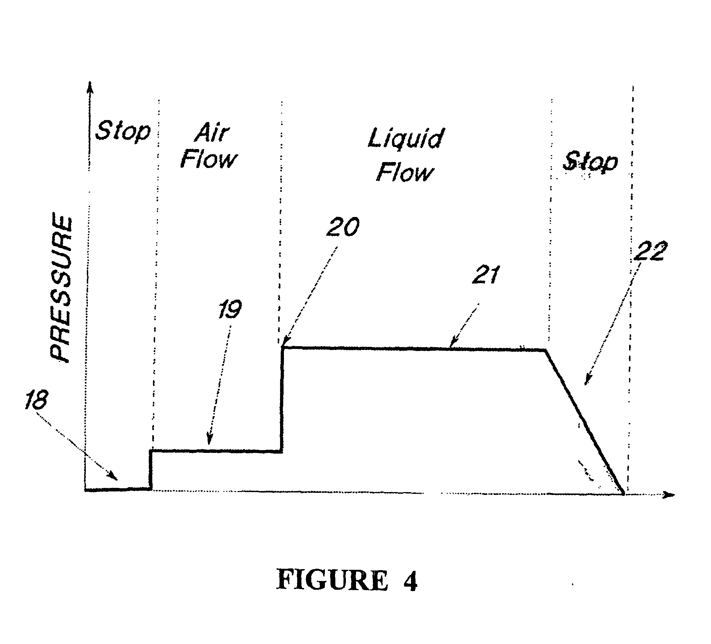

Figure 4 illustrates a chart of the pressure inside the tube of a flow set during a priming procedure. This figure will be described with reference to Figure 1. When a flow set **10** is to be installed in a liquid pump, the flow set **10 is** empty and the liquid dose not start to flow yet. Therefore, the pressure **18** in the tubes is low. When liquid starts to flow into the administration tubes, the liquid pushes the air from the tubes out through the anti-free-flow valve **16,** which has a resistance to the air flow and therefore the pressure rises to a medium level **19.** The liquid fills the whole flow set **10** and liquid starts to flow through the anti-free-flow valve **16.** The resistance of the valve to a liquid flow is significantly higher than the resistance to an air flow. A significant rise of pressure **20** is sensed and remains high **21** as long as liquid flows through the set. When the pump stops, the pressure slides down **21** to a low pressure. The significant rise of pressure **20** is used by the liquid pump to recognize that the flow set **10** is full. The pump controller compares the volume of liquid used to fill the flow set **10** with the inside volume of the flow set and uses this information to identify the flow set. If the liquid pump does not identify the installed flow set as the required flow set, the liquid pump stops operating or sets an alarm or both.

Although the invention has been described in conjunction with specific embodiments thereof, it is evident that many alternatives, modifications and variations will be apparent to those skilled in the art, accordingly, it is intended to embrace all such alternatives, modifications and variations that fall within scope of the appended claims.

## Claims

1. A disposal flow set (10) comprising:
(a) a drip chamber (11) or a spike;
(b) a first administration tube (12) to administer liquid from said drip chamber (11) as said spike;
(c) at least one pumping tube-segment (13), connected to said first administration tube (12), enabling said flow set to be installed in a liquid pump;
(d) a second administration tube (15) installed at the end of the last one of said pumping tube-segments (13); and
(e) an anti-free-flow valve (16) installed at the end of said second administration tube (15);
**characterized in that** the disposal flow set further includes:
(f) at least one identifying-key (24), whereby the or each pumping tube-segment (13) has a respective identifying-key (24) connected thereto and wherein each identifying-key (24) comprises a hoop that is clamped on said respective tube-segment and includes:
(i) a number of teeth (24a) with a unique combination of location and width, said teeth (24a) are to be inserted into a compatible niches (17a) in a specific liquid pump (17) and prevent insertion of said flow set to a non compatible liquid pump; and
(ii) a pressing-plate (24b) for being pressed by a door (25) of a liquid pump (17) in order to allow said tube-segment (13) to be pressed against a pressure sensor (26) of said liquid pump while the door of said liquid pump is closed enabling said liquid pump to use the sensed pressure to verify the existence of said flow set.

2. The disposal flow set of claim 1 wherein each pumping tube-segment (13) is bordered by a stopper (14) in both ends, said stoppers (14) are shaped to be installed in fitted niches in a liquid pump.

3. The disposal flow set of claim 1 wherein the length of each pumping tube-segment (13) is between 81 Millimetres and 83 Millimetres.

4. The disposal flow set of claim 1 wherein the insider diameter of said pumping tube-segments (13) is between 2.5 Millimetres and 2.7 Millimetres and the diameter of said first and/or second administration tube (12,15) is between 1.1 Millimetres and 1.3 Millimetres.

5. The disposal flow set of claim 1, further includes a controlling means in the inlet of said drip chamber (11), enabling the control of the volume of the entering drops to said drip chamber.

## Patentansprüche

1. Einmalflüssigkeitszufuhrset (10), umfassend:
(a) eine Tropfkammer (11) oder Spike,
(b) einen ersten Zufuhrschlauch (12) zum Verabreichen von Flüssigkeit aus der genannten Tropfkammer (11) oder dem genannten Spike,
(c) mindestens ein Pumpschlauchsegment (13), das mit dem genannten ersten Zufuhrschlauch (12) verbunden ist und das Einsetzen des genannten Flüssigkeitszufuhrsets in eine Flüssigkeitspumpe ermöglicht,
(d) einen zweiten Zufuhrschlauch (15), der am Ende des letzten der genannten Pumpschlauchsegmente (13) angebracht ist, und
(e) ein Ventil (16) gegen freien Durchfluss, das am Ende des genannten zweiten Zufuhrschlauches (15) angebracht ist,
**dadurch gekennzeichnet, dass** das Einmalflüssigkeitszufuhrset außerdem umfasst:
(f) mindestens einen Kennungsschlüssel (24), wobei das oder jedes Pumpschlauchsegment (13) über einen mit ihm verbundenen entsprechenden Kennungsschlüssel (24) verfügt und wobei jeder Kennungsschlüssel (24) einen Ring aufweist, der auf das genannte entsprechende Schlauchsegmente geklemmt ist und umfasst:
(i) eine Anzahl an Zähnen (24a) mit einer einmaligen Kombination von Anordnung und Breite, wobei die genannten Zähne (24a) in kompatible Vertiefungen (17a) in einer bestimmten Flüssigkeitspumpe (17) eingesetzt werden müssen und verhindern, dass das genannte Flüssigkeitszufuhrset in eine nicht kompatible Flüssigkeitspumpe eingesetzt wird, und
(ii) eine Druckplatte (24b), auf die eine Tür (25) einer Flüssigkeitspumpe (17) drücken soll, um zu ermöglichen, dass das genannte Schlauchsegment (13) an einen Drucksensor (26) der genannten Flüssigkeitspumpe gedrückt wird, während die Tür der genannten Flüssigkeitspumpe geschlossen wird, wodurch der genannten Flüssigkeitspumpe erlaubt wird, den festgestellten Druck zur Püfung der Anwesenheit des genannten Flüssigkeitszufuhrsets zu nutzen.

2. Einmalflüssigkeitszufuhrset nach Patentanspruch 1, in dem jedes Pumpschlauchsegment (13) an beiden Enden von einem Stopper (14) begrenzt wird, wobei die genannten Stopper (14) dafür geformt sind, in passende Vertiefungen in einer Flüssigkeitspumpe eingesetzt zu werden.

3. Einmalflüssigkeitszufuhrset nach Patentanspruch 1, in dem die Länge jedes Pumpschlauchsegments (13) zwischen 81 Millimetern und 83 Millimetern beträgt.

4. Einmalflüssigkeitszufuhrset nach Patentanspruch 1, in dem der Innendurchmesser der genannten Pumpschlauchsegmente (13) zwischen 2,5 Millimetern und 2,7 Millimetern beträgt und der Durchmesser des genannten ersten und/oder zweiten Zufuhrschlauches (12, 15) zwischen 1,1 Millimetern und 1,3 Millimetern.

5. Das Einmalflüssigkeitszufuhrset nach Patentanspruch 1 umfasst außerdem ein Regulierungsmittel am Zulauf der genannten Tropfkammer (11), das die Regulierung des Volumens der in die genannte Tropfkammer eintretenden Tropfen ermöglicht.

## Revendications

1. Ensemble de tuyaux jetables (10) comprenant :
(a) une chambre de goutte-à-goutte (11) ou un spike ;
(b) un premier tuyau d'administration (12) pour administrer du liquide à partir de la ladite chambre de goutte-à-goutte (11) ou dudit spike ;
(c) au moins un segment de tuyau de pompage (13) relié audit premier tuyau d'administration (12) qui permet audit ensemble de tuyaux d'être installé dans une pompe à liquide ;
(d) un second tuyau d'administration (15) installé à l'extrémité du dernier segment de tuyau de pompage (13) et
(e) une valve anti flux libre (16) installée à l'extrémité dudit second tuyau d'administration (15),
**caractérisé en ce que** l'ensemble de tuyaux jetables comprend de plus :
(f) au moins une clé d'identification (24), le ou chaque segment de tuyau de pompage (13) ayant une clé d'identification respective (24) qui lui est reliée et dans lequel chaque clé d'identification (24) comprend un arceau qui est serré sur ledit segment de tuyau respectif et qui comprend :
(i) un nombre de dents (24a) avec une combinaison unique d'emplacement et de largeur, lesdites dents (24a) devant être insérés dans un rentrant compatible (17a) dans une pompe à fluide spécifique (17) et empêchant l'insertion dudit ensemble de tuyaux dans une pompe à liquide non compatible et
(ii) une plaque de pression (24b) étant pressée par une porte (25) d'une pompe à liquide (17) pour permettre audit segment de tuyau (13) d'être pressé contre un détecteur de pression (26) de ladite pompe à liquide tandis que la porte de ladite pompe à liquide est fermée en permettant à ladite pompe à liquide d'utiliser la pression détecter pour vérifier l'existence dudit ensemble de tuyaux.

2. Ensemble de tuyaux selon la revendication 1 dans lequel chaque segment de tuyau de pompage (13) est bordé par un bouchon (14) dans les deux extrémités, lesdits bouchons (14) étant formés pour être installés dans des rentrants rapportés dans une pompe à liquide.

3. Ensemble de tuyaux selon la revendication 1 dans lequel la longueur de chaque segment de tuyau de pompage (13) est entre 81 et 83 millimètres.

4. Ensemble de tuyaux selon la revendication 1 dans lequel le diamètre intérieur desdits segments de tuyau de pompage (13) est entre 2,5 et 2,7 millimètres et le diamètre dudit premier et/ou dudit second tuyau d'administration (12, 15) est entre 1,1 et 1,3 millimètre.

5. Ensemble de tuyaux selon la revendication 1 comprenant de plus un moyen de contrôle dans l'entrée de ladite chambre de goutte-à-goutte (11) permettant le contrôle du volume des gouttes qui entrent dans ladite chambre goutte-à-goutte.
